# EUROPEAN PATENT SPECIFICATION

(11) **EP 4 567 152 B1**
(45) Date of publication and mention of the grant of the patent: **20.05.2026**
(21) Application number: 23215275.1
(22) Date of filing: 08.12.2023
(51) Int. Cl.: C23F 4/04, C25F 3/14, G01N 27/02, H01L 21/3213, G01N 33/00

(54) **METHOD FOR MANUFACTURING ELECTRODES AND ELECTRODES MANUFACTURED BY THIS METHOD**
VERFAHREN ZUR HERSTELLUNG VON ELEKTRODEN UND ELEKTRODEN HERGESTELLT NACH DIESEM VERFAHREN
PROCÉDÉ DE FABRICATION D'ÉLECTRODES ET ÉLECTRODES PRODUITES PAR CE PROCÉDÉ

(43) Date of publication of application: 11.06.2025
(73) Proprietor: IMEC VZW, 3001 Leuven (BE)
(72) Inventor: Fondu, Jelle, 3010 Kessel-Lo (BE); Severi, Simone, 3000 Leuven (BE)
(74) Representative: Patent Department IMEC

(56) References cited:
- WO-A1-2009/010389
- US-A1- 2021 288 152
- YUSUKE SHIOMI ET AL: "High-Yield Bridged Assembly of ssDNA-Modified SWCNT Using Dielectrophoresis", INTERNATIONAL JOURNAL OF AUTOMATION TECHNOLOGY, 5 January 2018 (2018-01-05), pages 29 - 36, XP055740241, Retrieved from the Internet <URL:https://www.fujipress.jp/ijat/au/ijate001200010029/> DOI: 10.20965/ijat.2018.p0029
- HINTSCHE R ET AL: "Microelectrode arrays and application to biosensing devices", BIOSENSORS AND BIOELECTRONICS, ELSEVIER SCIENCE LTD, UK, AMSTERDAM , NL, vol. 9, no. 9-10, 1 January 1994 (1994-01-01), pages 697 - 705, XP026594166, ISSN: 0956-5663, [retrieved on 19940101], DOI: 10.1016/0956-5663(94)80068-5

## Description

### Technical field

The present invention is generally related to a method of manufacturing a plurality of electrodes and more specifically to a method of manufacturing a plurality of electrodes comprising noble metal.

### Technical background

Noble metals, such as gold, platinum, and silver, are often used as electrode materials due to their high conductivity, chemical stability, and resistance to oxidation and corrosion. These properties make them ideal for use in a variety of applications, including electrochemical actuator, batteries and fuel cells. Electrical leakage is an unintended flow of electrical current between the electrodes, leading to a loss of efficiency and accuracy in the device. Electrical leakage can be caused by a variety of factors, including the presence of impurities or defects in the electrode material, or the formation of conductive pathways between the electrodes due to the accumulation of ions or other charged species. One issue that can arise when using noble metal electrodes is the problem of electrical leakage due to the re-sputtering of the noble metal particles in conventional manufacturing methods for electrode array in low pitch. Conventional etching methods for noble metals, such as gold, platinum, and silver, can have drawbacks, including the re-sputtering of metal particles. During the etching process, high-energy ions can cause the metal particles to be ejected from the surface, leading to re-sputtering. This can result in the formation of conductive pathways between the electrodes, increasing the risk of electrical leakage.

A method for manufacturing electrodes is known from the article Yusuke Shiomi ET AL: "High-Yield Bridged Assembly of ssDNA-Modified SWCNT Using Dielectrophoresis", International journal of automation technology, 5 January 2018, pages 29-36.

When the distance between electrodes gets smaller using conventional manufacturing methods, the risk of electrical leakage increases tremendously. This is because the smaller distance between the electrodes can make it easier for conductive pathways to form, allowing current to flow between the electrodes.

### Summary of the invention

The invention is set out in the appended set of claims.

It is an objective of the present disclosure to provide reliable methods for making electrodes comprising noble metal with low leakage between the electrodes. In the present disclosure, low leakage requires the resistance between electrodes to be at least 1e8 ohm. With the methods in the present disclosure, the distance between the two neighboring electrodes can be advantageously scaled to not larger than 500nm, preferably not larger than 300nm, more preferably not larger than 200nm, and more preferably not larger than 150nm. To minimize the risk of electrical leakage in devices with such small pitch sizes, it is important to carefully control the manufacture of the electrodes to prevent the buildup of conductive particles, e.g. metal particles, between the electrodes.

Reducing the effect of re-sputtering is important for eliminating electrical leakage, which is crucial for using such electrodes in bio-sensing and bio-actuating in liquid environments. To minimize the risk of electrical leakage, it is important to carefully control the etching process and use methods that reduce the impact of re-sputtering.

It is advantage of the present disclosure that economically efficient methods are provided for making an electrode array having a pitch not larger than 500nm, preferably not larger than 300nm, more preferably not larger than 200nm, and more preferably not larger than 150nm. Semiconductor device pitch is defined as the combined length of a single printed feature along with the adjacent space. In the present disclosure, this is equivalent to the distance measured from the centerline of a space between two neighboring electrodes (or electrode columns) to the centerline of the next space adjacent to the next pair of electrodes. Essentially, it is the distance between the imaginary line that runs through the centers of spaces situated between each pair of neighbouring electrodes or electrode columns.

It is an advantage of the present disclosure that reliable methods are provided for making electrodes wherein the dimensions of the electrodes can be well controlled.

It is an advantage of the present disclosure that at least a thousand of, in preferred embodiment at least a million of electrodes can be manufactured.

In some embodiments, an advantage of using the further step of reactive ion etching is that the methods further disables the leakage path formed by re-sputtered noble metal particles between the electrodes.

In some embodiment, it is an advantage for the sacrificial part to comprise a protruded structure of the dielectric layer. These methods prevent the buildup of conductive particles, i.e. re-sputtered noble metal particles, between the electrodes during the ion-beam etching step.

### Brief description of the drawings

The disclosure will be further elucidated by means of the following description and the appended figures. Various exemplary embodiments are described herein with reference to the following figures, wherein like numeral denotes like entities. The figures described are schematic and are non-limiting. Further, any reference signs in the claims shall not be construed as limiting the scope of the present disclosure. Still further, in the different figures, the same reference signs refer to the same or analogous elements.

The terms "over" and "above" are used for position indication of layers and not necessarily for describing a direct contact of the layers. It is to be understood that the terms so used are interchangeable under appropriate circumstances. The term "on" is used for position indication of layers and describing a direct contact of the layers.

The term "top surface" is used as a reference for a certain surface. It is to be understood that the "top surface" can be a bottom surface in figures under appropriate circumstances, for example when the surface/stack is turned around.
**Figure 1a to 1h** show an example schematic illustration of a cross section of a plurality of stacks resulting from the steps of the process in a first example.
**Figures 2a to 2h** show an example schematic illustration of a cross section of a plurality of stacks resulting from the steps of the process in a second example.
**Figure 3** shows an example top-down schematic illustration of an electrode array resulting from the steps of the process in the present disclosure.
**Figure 4** shows an exemplar flow chart of the main steps of an embodiment of the manufacture method in the present disclosure.

### Detailed description

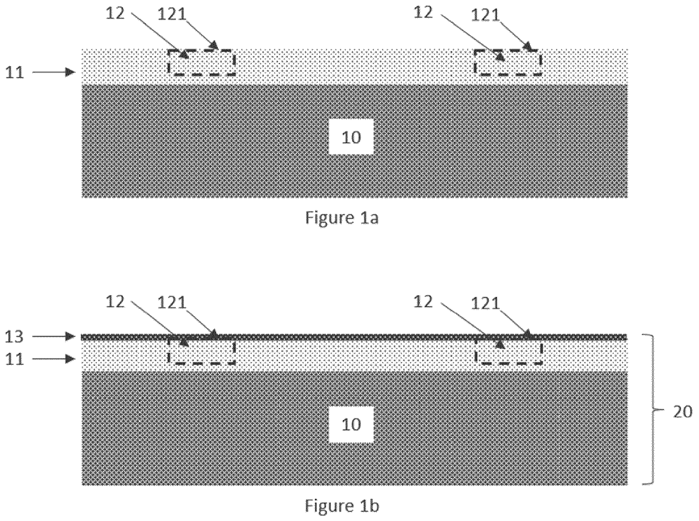

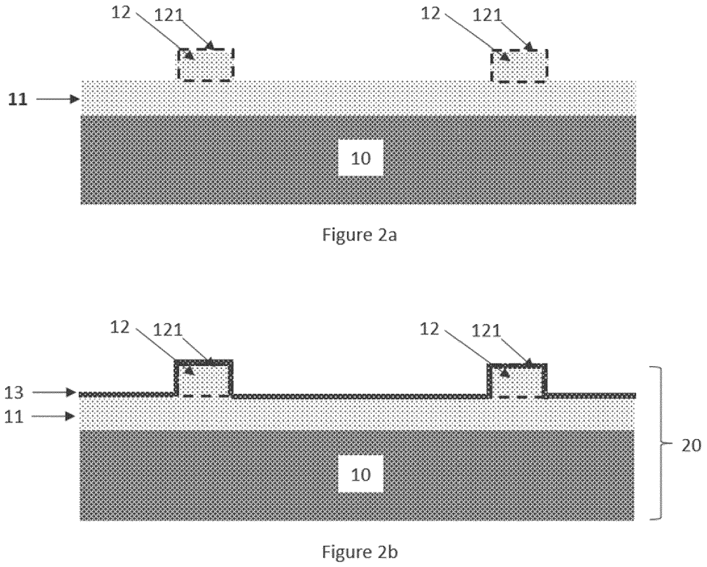

**The first aspect of the present disclosure** relates to the manufacture of a plurality of electrodes (15) wherein the electrodes (15) comprise noble metal as exemplified in Figure 1a to 1h, 2a to 2h. The method comprises the steps of:
a. providing a substrate (10) having a dielectric layer (11) in physical contact therewith, wherein the dielectric layer (11) comprises at least a sacrificial part (12), wherein the sacrificial part (12) comprises a top surface (121),
b. forming a conductive layer (13) on the dielectric layer (11) and in physical contact with the top surface (121) of the sacrificial part (12), wherein the conductive layer (13) comprises the noble metal, thereafter,
c. providing a masking layer (14) over the conductive layer (13), thereafter,
d. patterning the masking layer (14) to expose at least the conductive layer (13) above the sacrificial part (12) thereby defining a plurality of electrodes (15) wherein the smallest distance (d1) between the plurality of electrodes (15) is not larger than 500nm, preferably not larger than 300nm, more preferably not larger than 200nm, and more preferably not larger than 150nm, thereafter,
e. etching the conductive layer (13) by ion-beam thereby forming the plurality of electrodes (15) comprising the noble metal, thereafter,
f. etching at least a portion of the sacrificial part (12) by dry etching such that the resistance at the smallest distance (d1) between the plurality of electrodes (15) is at least 1e8 ohm,
g. removing the masking layer (14).

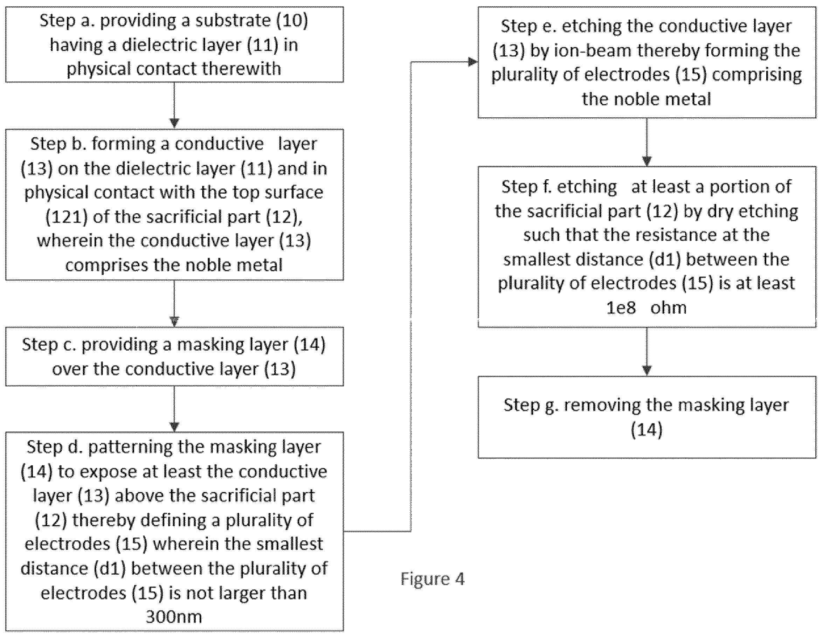

Figure 4 shows an exemplar flow chart of the main steps of an embodiment of the manufacture method in the present disclosure.

In some embodiments, the masking layer (14) is in physical contact with the conductive layer (13) in step c.

In some embodiments, step g of removing the masking layer (14) is directly after the step f of etching at least a portion of the sacrificial part (12) by dry etching.

In some embodiments, step g of removing the masking layer (14) is after the step e of etching the conductive layer (13) by ion-beam thereby forming the plurality of electrodes (15) and before step f of etching at least a portion of the sacrificial part (12) by dry etching, wherein the dry etching is a non-ion-beam etching.

In some embodiments, the step d of patterning the masking layer (14) to expose at least part of the conductive layer (13) above the sacrificial part (12) defines the edge of the plurality of electrodes (15) such that the smallest distance (d1) between the plurality of electrodes (15) is designed to be not larger than 500nm, preferably not larger than 300nm, more preferably not larger than 200nm, and more preferably not larger than 150nm.

In some embodiments, the masking layer (14) is formed as a conformal layer over the conductive layer (13).

In some embodiments, the sacrificial part (12) consist of the same material as in the dielectric layer (11) thus being part of the dielectric layer (11).

In some embodiments, the conductive layer (13) above and corresponding to the sacrificial part (12) is etched substantially completely by ion-beam thereby forming the plurality of electrodes (15) in step e.

The present disclosure concerns the manufacture of electrodes comprising at least one type of noble metal. An electrode is a conductor through which electric current can pass. Noble metals, such as gold, platinum, and silver, are often used as electrode materials due to their high conductivity, chemical stability, and resistance to oxidation and corrosion, even in liquid environment. These properties make them ideal for use in a variety of applications, including electrochemical actuator, batteries, and fuel cells.

In some embodiments, the noble metal is selected from the list consisting of platinum (Pt), gold (Au) or silver (Ag). In a preferred embodiment, the noble metal is platinum.

As shown in the Figures, the substrate (10) is a base material that supports the formation of other layers on top of it. In this application, the substrate is preferably composed of a semiconductor material, which is a type of material that can conduct electricity under certain conditions. For example, the substrate may be a circular wafer or a rectangular wafer die. In some embodiments, the substrate (10) comprises Silicon (Si), which is the most widely used material for integrated circuits and microelectronics devices. Silicon has high purity, low cost, and excellent electrical and thermal properties. The substrate can comprise other semiconductor materials such as Gallium arsenide (GaAs), Sapphire (Al₂O₃), Germanium (Ge).

In some embodiments, the substrate (10) comprises (e.g. consist of) semiconductor material. In some embodiment, the substrate (10) comprises (e.g. consist of) silicon.

In some embodiments, the substrate (10) comprises a stack of semiconductor layers. In some embodiments, the substrate (10) comprises a conductive layer for forming buried conductive connections between electrodes. In some embodiments, the substrate (10) comprises conductive layers and dielectric layers configured for forming switches or other control logic.

The substrate (10) has a dielectric layer (11) in physical contact therewith.

A dielectric layer (11) is a layer of material that has a high electrical resistance and can store electric charge. Dielectrics are often used as insulators or capacitors in electronic devices. The dielectric layer (11) may comprise any material that can be etched or dissolved by a chemical solution, such as silicon (di)oxide (e.g. SiO₂), silicon nitride (Si₃N₄), or polyimide. The dielectric layer (11) may have a thickness ranging from 10 nm to 10 µm. The dielectric layer (11) comprises a sacrificial part (12) that has a top surface (121). In some embodiments, the properties of the dielectric layer (11), such as thickness or material, are selected and designed such that the resistance between the electrodes (15) at the smallest distance can reach at least 1e8 ohm.

The dielectric layer (11) may either be an integral part of the substrate (10) or a separate layer deposited on one of the largest surfaces of the substrate (10) by any suitable method, such as chemical vapor deposition (CVD), physical vapor deposition (PVD), atomic layer deposition (ALD), or spin coating. In either case, the dielectric layer (11) is always in direct contact with the substrate. The dielectric layer (11) may have a uniform or non-uniform composition and thickness across the substrate (10). The dielectric layer (11) may also have different properties, such as refractive index, dielectric constant, or stress, depending on the application.

In some embodiments, the dielectric layer comprises (e.g. consist of) silicon oxide (e.g. SiₓO_{y}, such as SiO₂) and/or silicon nitride (e.g. SiₓN_{y}, such as Si₃N₄).

In some embodiments, the silicon nitride layer is deposited by chemical vapour deposition (CVD); e.g. plasma-enhanced CVD (PECVD) or low-pressure CVD (LPCVD).

In some embodiments, the silicon oxide is deposited by chemical vapour deposition (e.g. PECVD or LPCVD) or is formed by thermal oxidation of the Si substrate.

The sacrificial part (12) is a portion of the dielectric layer (11) that will be removed in a later step to further enhance the separation between electrodes (15).

The conductive layer (13) is a layer of electrically conductive material that is deposited on the dielectric layer (11) by any suitable method, such as sputtering, evaporation, electroplating, or electroless plating. The conductive layer (13) may have a uniform or non-uniform composition and thickness across the dielectric layer (11). The conductive layer (13) comprises at least one type of noble metal. The conductive layer (13) may comprise a single material, i.e. a noble metal or alloy thereof, or a combination of materials, such as with different type of noble metals, alloys. In the present disclosure, the conductive layer (13) must comprise a noble metal, such as platinum (Pt), gold (Au), silver (Ag) or palladium (Pd), which has high conductivity, low reactivity, and good compatibility with organic materials.

The top surface of the substrate (10) can be the top surface of a silicon wafer which is a substantially smooth and flat surface that serves as a base for, e.g. depositing various layers of materials. In an embodiment, the top surface of the substrate of a wafer may also have different patterns, such as trenches, holes, or pillars, that are formed by lithography and etching processes.

In some embodiments, the top surface of the substrate (10) is planarized by chemical mechanical polishing (CMP) before step b., which involves forming a conductive layer (13) on the dielectric layer (11) and in physical contact with the top surface (121) of the sacrificial part (12), wherein the conductive layer (13) comprises a noble metal.

In some embodiments, the top surface of the conductive layer (13) is planarized by chemical mechanical polishing (CMP) before step c., which involves providing a masking layer (14) in physical contact with the conductive layer (13).

In some embodiments, the masking layer comprises (e.g. consist of) Silicon Oxide (e.g. SiₓO_{y}, such as SiO₂), silicon nitride (e.g. SiₓN_{y}, such as Si₃N₄), Titanium (Ti), Titanium nitride (TiN) and/or diamond like carbon (DLC).

In some embodiments, the silicon nitride layer is deposited by chemical vapour deposition (e.g. PECVD or LPCVD).

In some embodiments, the silicon oxide is deposited by chemical vapour deposition (e.g. PECVD or LPCVD).

In some embodiments, the Ti or TiN is deposited by chemical vapour deposition (e.g. PECVD or LPCVD) or atomic layer deposition (ALD).

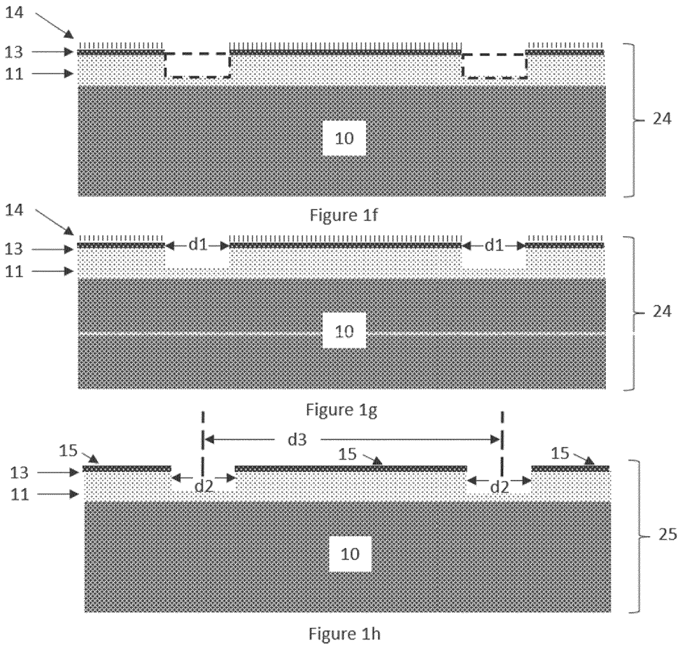

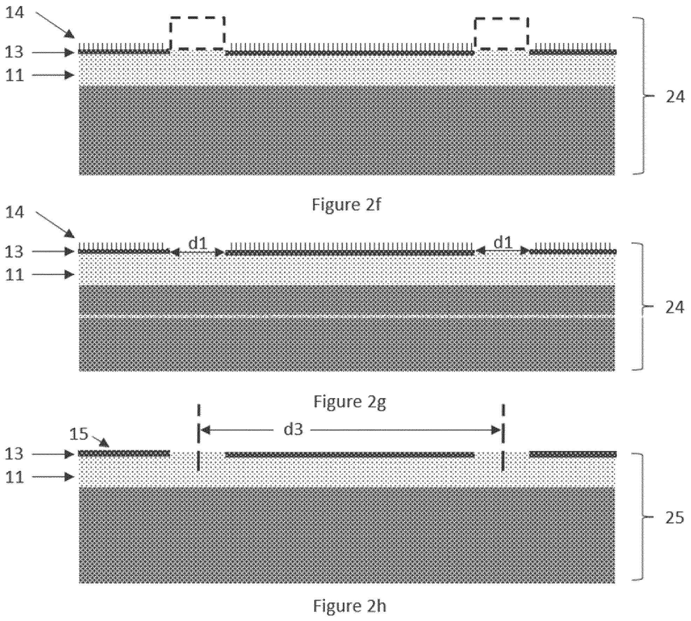

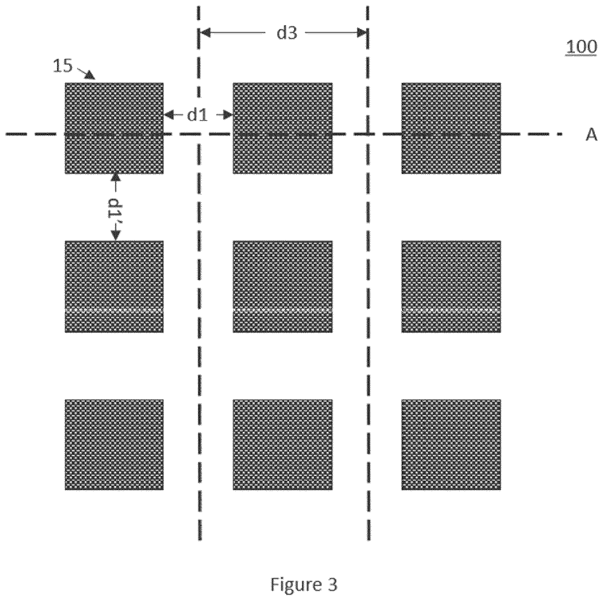

**The second aspect of the present disclosure** relates to an electrode array (100), as exemplified in Figure 3, obtained by the method described in the present disclosure. Figure 1h or Figure 2h in the first and second examples of the first aspect can be a zoom-in cross section along the indication line A in Figure 3.

In some embodiments, the array has a pitch distance (d1) of not larger than 500nm, preferably not larger than 300nm, more preferably not larger than 200nm, and more preferably not larger than 150nm. The pitch distance can be measured between a pair of neighboring electrodes, e.g. in the direction of indication line A (d1) or in the direction perpendicular to the indication line A (d1'). In an embodiment, the distance between the pair of neighboring in the direction of indication line A (d1) is substantially equal to the distance between the pair of neighboring in the direction perpendicular to indication line A (d1').

In some embodiments, the array comprises at least a thousand, preferably a million electrodes.

**The third aspect of the present disclosure** relates to an electroactive device for bio-material processing comprising:
the electrode array obtained by the method described in the present disclosure.
a reservoir for containing the bio-material in an electrolyte during the device's operation.
wherein the electrode array is arranged such that the electrode array is exposed to the electrolyte during the device's operation.

In some embodiments, the bio-material is biomolecules such as proteins, carbohydrates, lipids, and nucleic acids (e.g. DNA, RNA).

In some embodiments, the bio-material is biologic tissues such as cells and organoids.

In some embodiments, the electroactive device is configured for DNA or RNA synthesis on electrodes.

### Example 1

As shown in **Figure 1a****,** the substrate (10) is provided having a dielectric layer (11). Sacrificial part (12) is indicated in the dashed square. The sacrificial part (12) is an integral part of the dielectric layer (11).

The top surface of the dielectric layer (11) is planar and the top surface (121) of the sacrificial part (12) is coplanar with a directly surrounding top surface of the dielectric layer (11).

In some embodiments, the top surface of the dielectric layer (11) is planarized by Chemical Mechanical Polishing (CMP) before step b. of forming a conductive layer (13) on the dielectric layer (11) and in physical contact with the top surface (121) of the sacrificial part (12), wherein the conductive layer (13) comprises a noble metal.

As shown in Figure 1b, the conductive layer (13) is deposited directly on the dielectric layer (11). Thus, a stack (20) comprising the substrate (10), the dielectric layer (11), and the conductive layer (13) is formed after step b and before step c.

In some embodiments, the conductive layer (13) completely covers the top surface (121) of the sacrificial part (12), as shown in Figure 1b. The conductive layer (13) may have a thickness ranging from 10 nm to 100 nm, preferably from 10 nm to 20 nm.

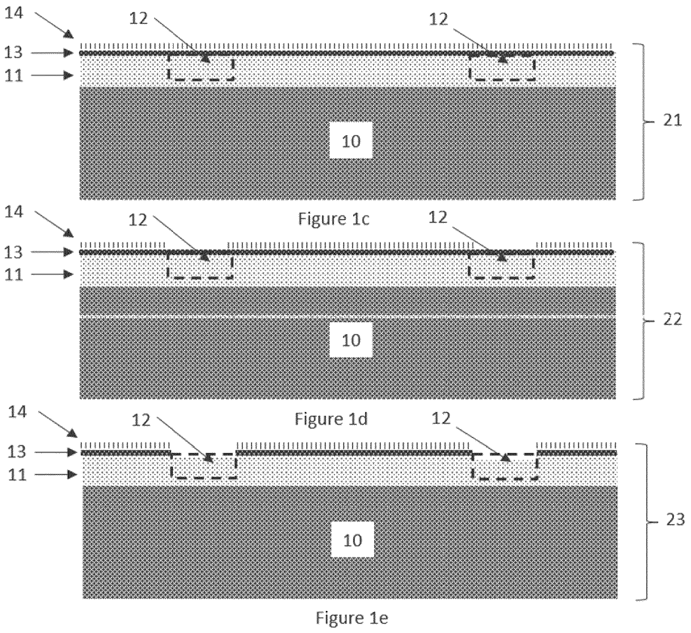

As shown in Figure 1c, the masking layer (14) is deposited directly on the conductive layer. Thus, a stack (21) comprising the substrate (10), the dielectric layer (11), the conductive layer (13) and the masking layer is formed after step c and before step d.

The masking layer (14) is provided in physical contact with the conductive layer (13), as shown in Figure 1c. In an embodiment, the dielectric layer, serving as a masking layer (14), is deposited over the conductive layer (13). The masking layer (14) completely covers the conductive layer (13) in the region corresponding to the sacrificial part (12). In an embodiment, the masking layer (14) can be made of a composite material comprising SiO₂, Si₃N₄, Ti and/or TiN. The masking layer (14) can be deposited by any suitable technique, such as chemical vapor deposition (CVD), atomic layer deposition (ALD), physical vapor deposition (PVD), or sputtering. The masking layer (14) can have a thickness ranging from 10 nm to 10 µm, more preferably 5nm to 1 µm.

As shown in Figure 1d, the masking layer (14) is patterned. The patterning of the masking layer (14) would expose at least part of the conductive layer to be etched, thereby defining the boundaries of the electrodes. A stack (22) comprising the substrate (10), the dielectric layer (11), the conductive layer (13) and a patterned masking layer is formed after step d and before step e.

The masking layer (14) is patterned to expose at least part of the conductive layer (13) in the regions corresponding to the sacrificial part (12). In an embodiment, the masking layer (14) is patterned to expose at least 70% of the conductive layer (13) in the regions corresponding to the sacrificial part (12). In an embodiment, the masking layer (14) is patterned to expose completely the conductive layer (13) in the regions corresponding to the sacrificial part (12). The patterning can be performed by any suitable technique, such as photolithography.

As shown in Figure 1e, the exposed part of the conductive layer (13) is etched by ion-beam etching. The ion-beam etching is a directional process that can effectively remove the conductive material. In this example, ion-beam etching uses Ar, Xe, Ne or Kr (i.e. the noble inert gases): this enables physical etching or sputtering and allows, among other processes, etching of noble metals such as Au, Pt, Pd which are non-reactive materials and hence do not respond to reactive plasma or chemical etching. The ion-beam etching can have an etching rate ranging from 1 nm/min to 100 nm/min depending on the ion source, the voltage, and the angle of incidence. The ion-beam etching can also be controlled by adjusting the duration and the area of exposure. The ion-beam etching removes the conductive layer (13) to form electrodes (15) on the dielectric layer (11) in the regions not corresponding to the sacrificial part (12). A stack (23) comprising the substrate (10), the dielectric layer (11), a patterned conductive layer (13), i.e. the electrodes (15), and a patterned masking layer (14) is formed after the step e and before step f.

The dielectric layer (14) serves as a masking layer to protect the conductive layer (13) from being removed in the regions not corresponding to the sacrificial part (12). However, the ion-beam etching will consume part of the masking layer (14). Thus, the masking layer (14) shall have a thickness and characteristics such that it is not completely removed before the conductive layer in the regions corresponding to the sacrificial part (12) is removed. In an embodiment, the masking layer shall have at least a thickness of 25% of the thickness before ion-beam etching. This can ensure that the electrodes (15) are well defined and aligned with the opening (13). Alternatively, the masking layer (14) can have a higher etching selectivity than the conductive layer (13), i.e. a lower etching rate, so that it can withstand the ion-beam etching longer than the conductive layer (13). In an embodiment, the conductive layer corresponding to the sacrificial part (12) is completely removed, ensuring that no electrodes will be formed on top of the sacrificial part (12).

As a side effect of the ion-beam etching, some noble metal particles are generated from the conductive layer (13) and re-sputtered in the space between the electrodes (15), i.e. above or on the surface of the sacrificial part (12). These noble metal particles can affect the electrical characteristics and performance of the device. Conventionally, a cleaning process can be performed to remove the re-sputtered noble metal particles from the space between the electrodes (15) by using a solvent, such as acetone, isopropanol, or ethanol, and applying ultrasonic waves to dissolve and dislodge the noble metal particles. However, the cleaning process using a solvent and ultrasonic wave can have some drawbacks. For example, the solvent can penetrate into the space between the electrodes (15) and the dielectric layer (11), which can cause swelling or cracking of the dielectric layer (11). Moreover, the solvent can also react with the dielectric layer (11) or the electrodes (15), which can degrade their properties or cause corrosion. Furthermore, the ultrasonic waves can generate mechanical stress or vibration on the electrodes (15), which can damage or detach them from the dielectric layer (11). Therefore, the conventional cleaning process using a solvent and ultrasonic wave can compromise the quality and reliability of the device. Furthermore, noble metal, such as Pt, is not removable by standard cleaning solvents. It is typically etched by aggressive solvent such as aqua regia.

As shown in Figure 1f, the sacrificial part (12) is etched by dry etching, e.g. reactive ion etching. The reactive ion etching can selectively remove the material of the sacrificial part (12) without damaging the electrodes (15). In some embodiments, the electrodes act as hard mask for reactive ion etching the sacrificial part (12).

It is better in this example if at least part of the step f of etching is done by a non-ion-beam dry etch so that further re-sputtering of metal particles is prevented. In an embodiment, part of the sacrificial part (12) is etched by ion-beam etching. Because of the non-ion-beam dry etch, the density of metal particles close to the edge of the electrode is significantly reduced thus increasing the resistance between the electrodes.

Thus, the resistance at the smallest distance (d1) between the plurality of electrodes (15), as indicated in Figure 1g, is at least 1e8 ohm.

The dry etching can have an etching rate ranging from 10 nm/min to 1000 nm/min depending on the material of the sacrificial part (12), the etching gas, and the etching parameters. The dry etching can also be controlled by adjusting the duration and the area of exposure. The dry etching removes at least a portion of the sacrificial part (12) to form a cavity that has a depth ranging from 10 nm to 200 nm.

In some embodiments, after the sacrificial part is etched, the dielectric layer (11) comprises recessed regions in the dielectric layer (11), corresponding to the removed sacrificial part, is obtained between the plurality of electrodes (15).

A stack (24) comprising the substrate (10), the dielectric layer (11) with recessed regions, a patterned conductive layer (13), i.e. the electrodes (15), and a patterned masking layer (14) is formed after the step f and before step g.

In some embodiments, as a last step, the masking layer (14) is removed by conventional etching methods. A stack (25) comprising the substrate (10), the dielectric layer (11) with recessed region and a patterned conductive layer (13), i.e. the electrodes (15) is formed after step f.

In some alternative embodiments, the masking layer (14) can be removed before the reactive ion etching of at least part of the sacrificial part (12).

As indicated in Figure 1g, the distance (d1) between the electrodes is defined in the step d of patterning the masking layer (14) to expose at least part of the conductive layer (13) above the sacrificial part (12). In an embodiment, the distance (d2) of the recessed region in the dielectric layer between the electrodes is substantively equal to the distance (d1) between the electrodes. Because, in some embodiments, the electrodes act as hard mask for reactive ion etching the sacrificial part (12). In an embodiment, the distance (d2) of the recessed region in the dielectric layer between the electrodes within 10% margin of the distance (d1) between the electrodes. In a preferred embodiment, the distance (d2) of the recessed region in the dielectric layer between the electrodes is within 5% margin of the distance (d1) between the electrodes.

The pitch (d3), as indicated in Figure 1h, is defined after step e of etching the conductive layer (13) by ion-beam thereby forming the plurality of electrodes (15).

The distance (d1) between the electrodes can be the smallest distance between any two electrodes of the plurality of electrodes. The resistance of the said two electrodes which has the smallest distance can be less than 1e8 ohm after dry etching step f.

To measure the resistance between the electrodes, the probes of the multimeter or the ohmmeter can be connected to the two electrodes whose resistance is to be measured.

### Example 2

As shown in **Figure 2a****,** the substrate (10) is provided having a dielectric layer (11). Sacrificial part (12) is indicated in the dashed square. The sacrificial part (12) is an integral part of the dielectric layer (11).

The sacrificial part (12) is a protruded structure (12) of the dielectric layer (11).

As shown in Figure 2b, the conductive layer (13) is deposited directly on the dielectric layer (11). Thus, a stack (20) comprising the substrate (10), the dielectric layer (11), the conductive layer (13) is formed after step b and before step c.

In some embodiments, the conductive layer (13) completely covers the top surface (121) of the sacrificial part (12), as shown in Figure 2b. The conductive layer (13) may have a thickness ranging from 10 nm to 100 nm, preferably from 10 nm to 20 nm.

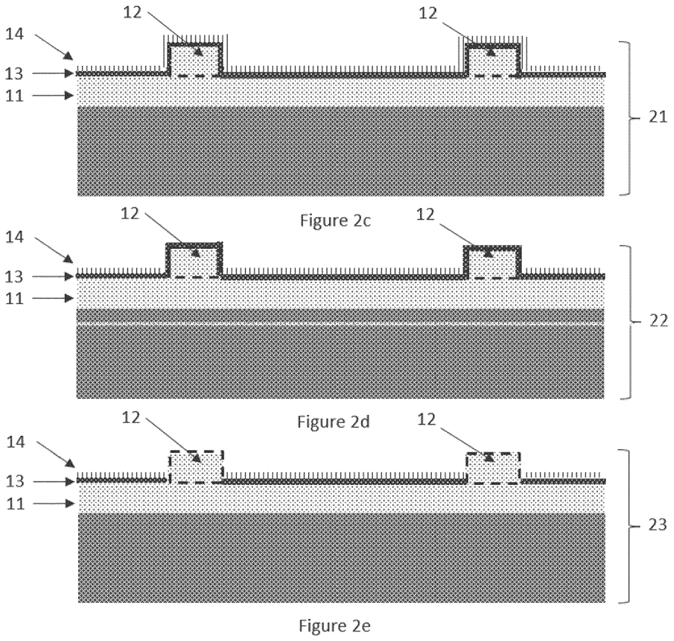

As shown in Figure 2c, the masking layer (14) is deposited directly on the conductive layer. Thus, a stack (21) comprising the substrate (10), the dielectric layer (11), the conductive layer (13) and masking layer is formed after step c and before step d.

The masking layer (14) is provided in physical contact with the conductive layer (13), as shown in Figure 2c. In an embodiment, a dielectric layer, serving as the masking layer (14), is deposited over the conductive layer (13). The masking layer (14) completely covers the conductive layer (13) in the region corresponding to the sacrificial part (12). In an embodiment, the masking layer (14) can be made of a composite material comprising SiO₂, Si₃N₄, Ti and/or TiN. The masking layer (14) can be deposited by any suitable technique, such as chemical vapor deposition (CVD), atomic layer deposition (ALD), physical vapor deposition (PVD), or sputtering. The masking layer (14) can have a thickness ranging from 10 nm to 10 µm, more preferably 5nm to 1 µm.

As shown in Figure 2d, the masking layer (14) is patterned. The patterning of the masking layer (14) would expose at least part of conductive layer to be etched thereby defining the boundary of the electrodes. A stack (22) comprising the substrate (10), the dielectric layer (11), the conductive layer (13) and a patterned masking layer is formed after step d and before step e.

The masking layer (14) is patterned to expose at least part of the conductive layer (13) above the protruded structure. In an embodiment, the masking layer (14) is patterned to expose at least 70% of the conductive layer (13) in the regions corresponding to the sacrificial part (12). In an embodiment, the masking layer (14) is patterned to expose completely the conductive layer (13) in the regions corresponding to the sacrificial part (12). The patterning can be performed by any suitable technique, such as photolithography. In an embodiment, the masking layer (14) is patterned to expose completely the conductive layer (13) in the regions corresponding to the sacrificial part (12) and a portion of the masking layer (14) in the neighboring regions of the regions corresponding to the sacrificial part (12).

As shown in Figure 2e, the exposed part of the conductive layer (13) is etched by ion-beam etching. The ion-beam etching is a directional process that can effectively remove the conductive material. In this example, ion-beam etching uses Ar, Xe, Ne or Kr (i.e. the noble inert gases): this enables physical etching or sputtering and allows, among other processes, etching of noble metals such as Au, Pt, Pd which are non-reactive materials and hence do not respond to reactive plasma or chemical etching. The ion-beam etching can have an etching rate ranging from 1 nm/min to 100 nm/min depending on the ion source, the voltage, and the angle of incidence. The ion-beam etching can also be controlled by adjusting the duration and the area of exposure. The ion-beam etching removes the conductive layer (13) to form electrodes (15) on the dielectric layer (11) in the regions not corresponding to the sacrificial part (12). A stack (23) comprising the substrate (10), the dielectric layer (11), a patterned conductive layer (13), i.e. the electrodes (15), and a patterned masking layer (14) is formed after step e and before step f.

The masking layer (14) protects the conductive layer (13) from being removed in the regions not corresponding to the sacrificial part (12). However, the ion-beam etching may consume the masking layer (14). Thus, the masking layer (14) shall have a thickness and characteristics such that it is not completely removed before the conductive layer in the regions corresponding to the sacrificial part (12) is removed. In an embodiment, the masking layer shall have at least a thickness of 25% of the thickness before ion-beam etching. This can ensure that the electrodes (15) are well defined and aligned with the opening (13).

Alternatively, the masking layer (14) can have a higher etching selectivity than the conductive layer (13), i.e. a lower etching rate, so that it can withstand the ion-beam etching longer than the conductive layer (13). In an embodiment, the conductive layer corresponding to the sacrificial part (12) is completely removed thus no electrode will be formed on top of the sacrificial part (12).

As a side effect of the ion-beam etching, some noble metal particles are generated from the conductive layer (13) and re-sputtered in the space between the electrodes (15), i.e. above or on the surface of the sacrificial part (12). These noble metal particles can affect the electrical characteristics and performance of the device. Conventionally, a cleaning process can be performed to remove the re-sputtered noble metal particles from the space between the electrodes (15) by using a solvent, such as acetone, isopropanol, or ethanol, and applying ultrasonic waves to dissolve and dislodge the noble metal particles. However, the cleaning process using a solvent and ultrasonic wave can have some drawbacks. For example, the solvent can penetrate into the space between the electrodes (15) and the dielectric layer (11), which can cause swelling or cracking of the dielectric layer (11). Moreover, the solvent can also react with the dielectric layer (11) or the electrodes (15), which can degrade their properties or cause corrosion. Furthermore, the ultrasonic waves can generate mechanical stress or vibration on the electrodes (15), which can damage or detach them from the dielectric layer (11). Therefore, the conventional cleaning process using a solvent and ultrasonic wave can compromise the quality and reliability of the device.

As shown in Figure 2f, the sacrificial part (12) is etched by dry etching.

In this example step f of etching can be done by any effective dry etch to remove the sacrificial part. Because of the existence of the protruded structure, the metal particles re-sputtered during the step e mainly lands on the masking layer. Much less of the metal particles would remain in the space between the electrodes.

In some embodiments, the dry etching in step f comprises a continuous ion-beam etching from step e. In other words, in some embodiments, the ion-beam etching initiated in step e continues in step f, thereby constituting the dry etching process for at least a portion of the sacrificial part in step f.

Thus, the resistance at the smallest distance (d1) between the plurality of electrodes (15), as indicated in Figure 1g, can be at least 1e8 ohm.

After the sacrificial part is etched, the dielectric layer (11) comprises regions corresponding to the removed sacrificial part.

In some embodiments, the sacrificial part (12) is further etched by non-ion-beam dry etching, e.g. reactive ion etching. The reactive ion etching can selectively remove the material of the sacrificial part (12) without damaging the electrodes (15). In some embodiments, the electrodes act as hard mask for reactive ion etching the sacrificial part (12).

Because of the non-ion-beam dry etch, the density of metal particles close to the edge of the electrode is further reduced thus increasing the resistance between the electrodes.

A stack (24) comprising the substrate (10), the dielectric layer (11), a patterned conductive layer (13), i.e. the electrodes (15), and a patterned masking layer (14) is formed after the step f and before step g.

In some embodiments, as a last step, the masking layer (14) is removed by conventional etching methods. A stack (25) comprising the substrate (10), the dielectric layer (11) and a patterned conductive layer (13), i.e. the electrodes (15) is formed after step f.

In some other embodiments, the masking layer (14) can be removed before the further non-ion-beam dry etching, e.g. reactive ion etching, by conventional etching methods.

As indicated in Figure 2g, the distance (d1) between the electrodes is defined in the step d of patterning the masking layer (14) to expose at least part of the conductive layer (13) above the sacrificial part (12).

The pitch (d3), as indicated in Figure 2h, is defined after step e of etching the conductive layer (13) by ion-beam thereby forming the plurality of electrodes (15).

The distance (d1) between the electrodes can be the smallest distance between any two electrodes of the plurality of electrodes. The resistance of the said two electrodes which has the smallest distance can be less than 1e8 ohm after dry etching step f.

In some embodiment, the dry etching in step f comprises a further step of non-ion-beam etching, e.g. reactive ion etching.

The dry etching can have an etching rate ranging from 10 nm/min to 1000 nm/min depending on the material of the sacrificial part (12), the etching gas, and the etching parameters. The dry etching can also be controlled by adjusting the duration and the area of exposure. The dry etching removes at least a portion of the sacrificial part (12) to form a cavity (17) that has a depth ranging from 10 nm to 200 nm.

After the sacrificial part is etched, the dielectric layer (11) comprises recessed regions corresponding to the removed sacrificial part.

A stack (24) comprising the substrate (10), the dielectric layer (11) with recessed regions, a patterned conductive layer (13), i.e. the electrodes (15), and a patterned masking layer (14) is formed after the step f and before step g.

As a last step, the masking layer (14) is removed by conventional etching methods. A stack (25) comprising the substrate (10), the dielectric layer (11) with recessed region and a patterned conductive layer (13), i.e. the electrodes (15) is formed after step f.

As indicated in Figure 2g, the distance (d1) between the electrodes is defined in the step d of patterning the masking layer (14) to expose at least part of the conductive layer (13) above the sacrificial part (12). In an embodiment, the distance (d2) of the recessed region in the dielectric layer between the electrodes is substantively equal to the distance (d1) between the electrodes. In an embodiment, the distance (d2) of the recessed region in the dielectric layer between the electrodes is within 10% margin of the distance (d1) between the electrodes.

The pitch (d3), as indicated in Figure 2h, is defined after step e of etching the conductive layer (13) by ion-beam thereby forming the plurality of electrodes (15).

The distance (d1) between the electrodes can be the smallest distance between any two electrodes of the plurality of electrodes. The resistance of the said two electrodes which has the smallest distance can be less than 1e8 ohm after dry etching step f.

To measure the resistance between the electrodes, the probes of the multimeter or the ohmmeter can be connected to the two electrodes whose resistance is to be measured.

## Claims

1. Method for manufacturing a plurality of electrodes (15) wherein the electrodes (15) comprise noble metal, the method comprising:
a. providing a substrate (10) having a dielectric layer (11) in physical contact therewith,
wherein the dielectric layer (11) comprises at least a sacrificial part (12),
wherein the sacrificial part (12) comprises a top surface (121),
b. forming a conductive layer (13) on the dielectric layer (11) and in physical contact with the top surface (121) of the sacrificial part (12), wherein the conductive layer (13) comprises the noble metal, thereafter
c. providing a masking layer (14) over the conductive layer (13), thereafter
d. patterning the masking layer (14) to expose at least the conductive layer (13) above the sacrificial part (12) thereby defining a plurality of electrodes (15) wherein the smallest distance (d1, d1') between the plurality of electrodes (15) is not larger than 500 nm, thereafter
e. etching the conductive layer (13) by ion-beam thereby forming the plurality of electrodes (15) comprising the noble metal, thereafter
f. etching at least a portion of the sacrificial part (12) by dry etching such that the resistance at the smallest distance (d1, d1') between the plurality of electrodes (15) is at least 1e8 ohm,
g. removing the masking layer (14).

2. The method according to claim 1, wherein the sacrificial part (12) comprises a protruded structure (122) of the dielectric layer (11).

3. The method according to claim 2, wherein the step f of etching at least a portion of the sacrificial part (12) by dry etching such that the resistance at the smallest distance (d1, d1') between the plurality of electrodes is at least 1e8 ohm, comprises a step of ion-beam etching.

4. The method according to claim 2 or 3, wherein the step f of etching at least a portion of the sacrificial part (12) by dry etching such that the resistance at the smallest distance (d1, d1') between the plurality of electrodes (15) is at least 1e8 ohm comprises a step of reactive ion etching.

5. The method according to claim 1, wherein a top surface of the dielectric layer (11) is planar and the top surface (121) of the sacrificial part (12) is coplanar with a directly surrounding top surface of the dielectric layer (11), wherein the step f of etching at least a portion of the sacrificial part (12) by dry etching such that the resistance at the smallest distance (d1, d1') between the plurality of electrodes (15) is at least 1e8 ohm comprises a step of reactive ion etching.

6. The method according to any of the preceding claims, wherein the noble metal is selected from the list consisting of platinum (Pt), gold (Au) and silver (Ag).

7. The method according to any of the preceding claims, wherein the dielectric layer (11) comprises SiO₂ and/or SiₓN_{y}.

8. The method according to any of the preceding claims, wherein the masking layer (14) comprises SiO₂, SiₓN_{y}, Ti, TiN and/or DLC.

9. The method according to any of the preceding claims, wherein step f of etching at least a portion of the sacrificial part (12) by dry etching such that the resistance at the smallest distance (d1) between the plurality of electrodes (15) is at least 1e8 ohm, is performed such that a recessed region in the dielectric layer (11) is obtained between the plurality of electrodes (15).

10. The method according to any of the preceding claims, wherein the sacrificial part (12) is recessed for at least 20nm with regard to the top surface (121) after the step f of etching at least a portion of the sacrificial part (12) by dry etching such that the resistance at the smallest distance (d1, d1') between the plurality of electrodes (15) is at least 1e8 ohm.

11. An electrode array obtained by the method according to any of the claims 1 to 10.

12. The electrode array according to claim 11 wherein the array has a pitch distance of not larger than 500nm.

13. The electrode array according to any of the claims 11 to 12 wherein the array comprises at least a thousand, preferably a million of electrodes.

14. The electrode array according to any of the claims 11 to 13 wherein the electrodes comprise platinum.

15. An electroactive device for bio-material processing comprising:
the electrode array according to any of the claim 11 to 13;
a reservoir for containing the bio-material in an electrolyte during the device's operation;
wherein the electrode array is arranged such that the electrode array is exposed to the electrolyte during the device's operation.

## Patentansprüche

1. Verfahren zur Herstellung einer Vielzahl von Elektroden (15), wobei die Elektroden (15) Edelmetall umfassen, wobei das Verfahren Folgendes umfasst:
a. Bereitstellen eines Substrats (10), das eine dielektrische Schicht (11) aufweist, die in physischem Kontakt damit steht,
wobei die dielektrische Schicht (11) mindestens einen Opferteil (12) umfasst,
wobei das Opferteil (12) eine obere Oberfläche (121) umfasst,
b. Bilden einer leitfähigen Schicht (13) auf der dielektrischen Schicht (11) und in physischem Kontakt mit der oberen Oberfläche (121) des Opferteils (12), wobei die leitfähige Schicht (13) das Edelmetall umfasst, danach
c. Bereitstellen einer Maskierungsschicht (14) über der leitfähigen Schicht (13), danach
d. Strukturieren der Maskierungsschicht (14), um mindestens die leitfähigen Schicht (13) über dem Opferteil (12) freizulegen, um dadurch eine Vielzahl von Elektroden (15) zu definieren, wobei der kleinste Abstand (d1, d1') zwischen der Vielzahl von Elektroden (15) nicht größer als 500 nm ist, danach
e. Ätzen der leitfähigen Schicht (13) durch Ionenstrahl, wodurch die Vielzahl von Elektroden (15) aus dem Edelmetall gebildet wird, danach
f. Ätzen mindestens eines Abschnitts des Opferteils (12) durch Trockenätzen, sodass der Widerstand bei dem kleinsten Abstand (d1, d1') zwischen der Vielzahl von Elektroden (15) mindestens 1e8 Ohm beträgt,
g. Entfernen der Maskierungsschicht (14).

2. Verfahren nach Anspruch 1, wobei das Opferteil (12) eine vorstehende Struktur (122) der dielektrischen Schicht (11) umfasst.

3. Verfahren nach Anspruch 2, wobei der Schritt f des Ätzens mindestens eines Abschnitts des Opferteils (12) durch Trockenätzen, sodass der Widerstand bei dem kleinsten Abstand (d1, d1') zwischen der Vielzahl von Elektroden mindestens 1e8 Ohm beträgt, einen Schritt des Ionenstrahlätzens umfasst.

4. Verfahren nach Anspruch 2 oder 3, wobei der Schritt f des Ätzens mindestens eines Abschnitts des Opferteils (12) durch Trockenätzen, sodass der Widerstand bei dem kleinsten Abstand Distanz (d1, d1') zwischen der Vielzahl von Elektroden (15) mindestens 1e8 Ohm beträgt, einen Schritt des reaktiven Ionenätzens umfasst.

5. Verfahren nach Anspruch 1, wobei die obere Oberfläche der dielektrischen Schicht (11) planar ist und die obere Oberfläche (121) des Opferteils (12) koplanar mit einer unmittelbar umgebenden oberen Oberfläche der dielektrischen Schicht (11) ist, wobei der Schritt f des Ätzens mindestens eines Abschnitts des Opferteils (12) durch Trockenätzen, sodass der Widerstand bei dem kleinsten Abstand (d1, d1') zwischen der Vielzahl von Elektroden (15) mindestens 1e8 Ohm beträgt, einen Schritt des reaktiven Ionenätzens umfasst.

6. Verfahren nach einem der vorstehenden Ansprüche, wobei das Edelmetall aus der Liste bestehend aus Platin (Pt), Gold (Au) und Silber (Ag) ausgewählt ist.

7. Verfahren nach einem der vorstehenden Ansprüche, wobei die dielektrische Schicht (11) SiO₂ und/oder SiₓN_{y} umfasst.

8. Verfahren nach einem der vorstehenden Ansprüche, wobei die Maskierungsschicht (14) SiO₂, SiₓN_{y}, Ti, TiN und/oder DLC umfasst.

9. Verfahren nach einem der vorstehenden Ansprüche, wobei Schritt f des Ätzens mindestens eines Abschnitts des Opferteils (12) durch Trockenätzen, sodass der Widerstand bei dem kleinsten Abstand (d1) zwischen der Vielzahl von Elektroden (15) mindestens 1e8 Ohm beträgt, so durchgeführt wird, dass ein vertiefter Bereich in der dielektrischen Schicht (11) zwischen der Vielzahl von Elektroden (15) erhalten wird.

10. Verfahren nach einem der vorstehenden Ansprüche, wobei das Opferteil (12) nach dem Schritt f des Ätzens mindestens eines Abschnitts des Opferteils (12) durch Trockenätzen um mindestens 20 nm in Bezug auf die obere Oberfläche (121) vertieft ist, sodass der Widerstand bei dem kleinsten Abstand (d1, d1') zwischen der Vielzahl von Elektroden (15) mindestens 1e8 Ohm beträgt.

11. Elektrodenanordnung, die nach dem Verfahren nach einem der Ansprüche 1 bis 10 erhalten wurde.

12. Elektrodenanordnung nach Anspruch 11, wobei die Anordnung einen Teilungsabstandnicht größer als 500 nm aufweist.

13. Elektrodenanordnung nach einem der Ansprüche 11 oder 12, wobei die Anordnung mindestens eintausend, vorzugsweise eine Million Elektroden umfasst.

14. Elektrodenanordnung nach einem der Ansprüche 11 bis 13, wobei die Elektroden Platin umfassen.

15. Elektroaktive Vorrichtung zur Verarbeitung von Biomaterialien, umfassend:
die Elektrodenanordnung nach einem der Ansprüche 11 bis 13;
einen Behälter zum Enthalten des Biomaterials in einem Elektrolyten während des Betriebs der Vorrichtung;
wobei die Elektrodenanordnung so angeordnet ist, dass die Elektrodenanordnung während des Betriebs der Vorrichtung dem Elektrolyten ausgesetzt ist.

## Revendications

1. Procédé de fabrication d'une pluralité d'électrodes (15), dans lequel les électrodes (15) comprennent un métal noble, le procédé comprenant :
a. la fourniture d'un substrat (10) présentant une couche diélectrique (11) en contact physique avec celui-ci,
dans lequel la couche diélectrique (11) comprend au moins une partie sacrificielle (12),
dans lequel la partie sacrificielle (12) comprend une surface supérieure (121),
b. la formation d'une couche conductrice (13) sur la couche diélectrique (11) et en contact physique avec la surface supérieure (121) de la partie sacrificielle (12), dans lequel la couche conductrice (13) comprend le métal noble, puis
c. la fourniture d'une couche de masquage (14) au dessue de la couche conductrice (13), puis
d. la structuration de la couche de masquage (14) pour exposer au moins la couche conductrice (13) au-dessus de la partie sacrificielle (12), définissant ainsi une pluralité d'électrodes (15), dans lequel la plus petite distance (d1, d1') entre la pluralité d'électrodes (15) ne dépasse pas 500 nm, puis
e. la gravure de la couche conductrice (13) par faisceau d'ions, formant ainsi la pluralité d'électrodes (15) comprenant le métal noble, puis
f. la gravure d'au moins une partie de la partie sacrificielle (12) par gravure sèche de sorte que la résistance à la plus petite distance (d1, d1') entre la pluralité d'électrodes (15) soit d'au moins 1e8 ohms,
g. la suppression de la couche de masquage (14).

2. Procédé selon la revendication 1, dans lequel la partie sacrificielle (12) comprend une structure saillante (122) de la couche diélectrique (11).

3. Procédé selon la revendication 2, dans lequel l'étape f de gravure d'au moins une partie de la partie sacrificielle (12) par gravure sèche de sorte que la résistance à la plus petite distance (d1, d1') entre la pluralité d'électrodes soit d'au moins 1e8 ohms, comprend une étape de gravure par faisceau d'ions.

4. Procédé selon la revendication 2 ou 3, dans lequel l'étape f de gravure d'au moins une partie de la partie sacrificielle (12) par gravure sèche de sorte que la résistance à la plus petite distance (d1, d1') entre la pluralité d'électrodes (15) soit d'au moins 1e8 ohms comprend une étape de gravure ionique réactive.

5. Procédé selon la revendication 1, dans lequel une surface supérieure de la couche diélectrique (11) est plane et la surface supérieure (121) de la partie sacrificielle (12) est coplanaire avec une surface supérieure directement environnante de la couche diélectrique (11), dans lequel l'étape f de gravure d'au moins une partie de la partie sacrificielle (12) par gravure sèche de sorte que la résistance à la plus petite distance (d1, d1') entre la pluralité d'électrodes (15) soit d'au moins 1e8 ohms comprend une étape de gravure ionique réactive.

6. Procédé selon l'une quelconque des revendications précédentes, dans lequel le métal noble est choisi dans la liste constituée du platine (Pt), de l'or (Au) et de l'argent (Ag).

7. Procédé selon l'une quelconque des revendications précédentes, dans lequel la couche diélectrique (11) comprend du SiO₂ et/ou du SiₓN_{y}.

8. Procédé selon l'une quelconque des revendications précédentes, dans lequel la couche de masquage (14) comprend du SiO₂, du SiₓN_{y}, du Ti, du TiN et/ou du DLC.

9. Procédé selon l'une quelconque des revendications précédentes, dans lequel l'étape f de gravure d'au moins une partie de la partie sacrificielle (12) par gravure sèche de sorte que la résistance à la plus petite distance (d1) entre la pluralité d'électrodes (15) soit d'au moins 1e8 ohms, est réalisée de manière à obtenir une région en retrait dans la couche diélectrique (11) entre la pluralité d'électrodes (15).

10. Procédé selon l'une quelconque des revendications précédentes, dans lequel la partie sacrificielle (12) est en retrait d'au moins 20 nm par rapport à la surface supérieure (121) après l'étape f de gravure d'au moins une partie de la partie sacrificielle (12) par gravure sèche de sorte que la résistance à la plus petite distance (d1, d1') entre la pluralité d'électrodes (15) soit d'au moins 1e8 ohms.

11. Réseau d'électrodes obtenu par le procédé selon l'une quelconque des revendications 1 à 10.

12. Réseau d'électrodes selon la revendication 11, dans lequel le réseau présente une distance de pas ne dépassant pas 500 nm.

13. Réseau d'électrodes selon l'une quelconque des revendications 11 à 12, dans lequel le réseau comprend au moins mille électrodes, de préférence un million d'électrodes.

14. Réseau d'électrodes selon l'une quelconque des revendications 11 à 13, dans lequel les électrodes comprennent du platine.

15. Dispositif électroactif pour le traitement de biomatériau comprenant :
le réseau d'électrodes selon l'une quelconque des revendications 11 à 13 ;
un réservoir destiné à contenir le biomatériau dans un électrolyte pendant le fonctionnement du dispositif ;
dans lequel le réseau d'électrodes est agencé de telle sorte que le réseau d'électrodes soit exposé à l'électrolyte pendant le fonctionnement du dispositif.
